# EUROPÄISCHE PATENTANMELDUNG

(11) **EP 0 530 820 A2**
(43) Veröffentlichungstag der Anmeldung: **10.03.1993**
(21) Anmeldenummer: 92115152.8
(22) Anmeldetag: 03.09.1992
(51) Int. Cl.: C12C 7/00, C12M 1/36, C12G 1/02

(54) **Verfahren und Vorrichtung zum Behandeln von Maische, insbesondere Rotwein-Maische**

(30) Priorität: 03.09.1991 DE 4129174
(71) Anmelder: RIEGER BEHÄLTERBAU GmbH, D-74321 Bietigheim-Bissingen (DE)
(72) Erfinder: Rieger, Herbert#, W-7121, Ingersheim (DE)
(74) Vertreter: Witte, Alexander, Dr.-Ing.

(57) **Zusammenfassung**

Ein Verfahren und eine Vorrichtung dienen zum Behandeln von Maische, insbesondere Rotweinmaische. Die Maische wird in einen Behälter (10) eingefüllt und mindestens ein physikalischer Parameter der Maische wird erfaßt. Ein Gärverlauf der Maische wird in Abhängigkeit von dem Parameter geregelt. Die Maische wird nach dem Druckentspannungsverfahren behandelt. Der Druck im Behälter (10) wird mittels eines Drucksensors (22) erfaßt und mittels eines Steuergerätes (30) über ein Druckentspannungsventil (24) des Behälters (10) geregelt (Fig. 1).

## Beschreibung

Die Erfindung betrifft ein Verfahren zum Behandeln von Maische, insbesondere Rotwein-Maische, bei dem die Maische in einen Behälter eingefüllt, mindestens ein physikalischer Parameter der Maische erfaßt und ein Gärverlauf der Maische in Abhängigkeit von dem Parameter geregelt wird.

Die Erfindung betrifft ferner eine Vorrichtung zum Durchführen des vorstehend genannten Verfahrens.

Es ist bekannt, bei der Behandlung von Maische, insbesondere von Rotwein-Maische die in einen Behälter eingefüllte Maische hinsichtlich ihrer Temperatur zu überwachen und die Maischetemperatur über geeignete Temperiereinheiten auf eine vorbestimmte Temperatur zu bringen oder dort zu halten. Hierzu verwendet man Temperaturregler mit einem Temperaturfühler, der die Maischetemperatur mißt. Über ein Steuergerät wird dann mittels eines Wärmetauschers ein Temperiermedium, üblicherweise Wasser oder Wasserdampf, zum Kühlen oder Wärmen der Maische eingesetzt. Dies geschieht üblicherweise mit Temperierschlangen, die in die Wandung des Behälters integriert oder als separate Elemente im Behälter vorgesehen sind, beispielsweise in den Armen eines Rührwerks.

Es ist ferner bekannt, zum Lösen der roten Farbstoffe aus den Schalen von Weinbeeren unterschiedliche Techniken einzusetzen. Eine dieser Techniken ist das sogenannte Druckentspannungsverfahren, das Anfang der 50er Jahre von Klenk entwickelt wurde.

Bei dem Druckentspannungsverfahren wird die frische, noch unvergorene Rotweinmaische in einen Druckbehälter eingefüllt und dieser Behälter dann druckdicht verschlossen. Falls erforderlich, wird die Maische auf Gärstarttemperatur gebracht, so daß die alkoholische Gärung einsetzen kann. Infolge der Gärung entwickelt sich im Behälter ein Überdruck, der über mehrere Stunden ansteigt. Mittels eines Manometers kann der Gärdruck abgelesen werden, bis ein vorbestimmter Abblasdruck erreicht ist. Mittels eines handbetätigten Ventils wird nun der Druck schlagartig entspannt, d.h. der Überdruck im Behälter abgebaut.

Die schlagartige Druckentspannung bewirkt, daß die Rotweinmaische von einer Druckwelle durcheilt wird, die eine mechanische Bewegung der Maische zur Folge hat. Insbesondere wird der sogenannte Tresterkuchen, d.h. die festen Bestandteile der Rotweinmaische, die sich aufgrund ihres geringeren spezifischen Gewichtes an der Oberfläche des Traubensaftes abgesetzt haben, aufgebrochen und überspült. Dies geschieht u.a. deswegen, weil der Traubensaft bei dem schlagartigen Druckentspannen zu sprudeln beginnt, indem Kohlensäure aus dem Traubensaft austritt, aufschäumt, den Tresterkuchen aufbricht und diesen überspült.

Nach erfolgter Druckentspannung wird das Abblasventil wieder geschlossen, so daß sich erneut Gärdruck aufbauen kann. Nach wiederum einigen Stunden ist der Abblasdruck erneut erreicht, so daß durch Öffnen des Abblasventiles wiederum eine Druckentspannung vollzogen werden kann.

Dieses Spiel wiederholt sich so oft, wie die Gäraktivität der Rotweinmaische ausreicht, um immer wieder den erforderlichen Abblasdruck im Behälter aufzubauen.

Das bekannte Verfahren hat jedoch den Nachteil, daß eine sehr aufwendige Überwachung des Gärverlaufes notwendig ist, weil sich der Gärverlauf nicht exakt voraussagen läßt und man daher nicht im voraus bestimmen kann, wann das Abblasventil geöffnet werden soll. Dies hat zur Folge, daß derartige Verfahren eine Überwachung des Behälters rund um die Uhr voraussetzen, damit jeweils bei Erreichen des Abblasdrucks gewährleistet ist, daß eine Druckentspannung vorgenommen wird. Wird nämlich der Druck im Behälter nicht rechtzeitig entspannt, so kann es ohne weiteres zum Bersten des Behälters kommen, weil die Gäraktivität von Rotweinmaische durchaus ausreicht, um Gärdrücke zu erzeugen, denen übliche Druckbehälter nicht standhalten.

Es ist daher u.a. sehr personalaufwendig, nach dem Druckentspannungsverfahren zu arbeiten, weil der oder die Behälter Tag und Nacht überwacht werden muß.

Das bekannte Verfahren hat ferner den Nachteil, daß es wenig differenziert arbeitet, weil üblicherweise nach bestimmten Faustregeln nur ein Abblasdruck festgelegt und die Maische dann beim Erreichen des Abblasdrucks entspannt wird. Eine differenzierte Behandlung von unterschiedlichen Rebsorten oder unterschiedlichen Jahrgängen oder gar eine Variation der Maischebehandlung nach persönlichen geschmacklichen Gesichtspunkten ist mit dem bekannten Druckentspannungsverfahren nicht möglich.

Es sind ferner unterschiedliche Verfahren bekannt, um die Gärung von Maische im Handbetrieb zu führen, indem beispielsweise durch Probenentnahme oder sonstiges Beobachten der gärenden Maische der Zustand der Maische von Zeit zu Zeit überprüft und dann von Hand ein Parameter der Maische nachgestellt wird, beispielsweise die Temperatur eines Wärmetauschers. Es ist ferner bekannt, die vergorene Maische weiteren Prüfungen zu unterziehen und dann die Bearbeitung fortzusetzen, z.B. zum Zwecke eines biologischen Säureabbaus.

Auch diese bekannten manuellen Verfahren haben den Nachteil, daß eine Überprüfung bzw. Beobachtung der Maische stets nur von Zeit zu Zeit vorgesehen ist und daß eine Einflußnahme auf den Gärverlauf mühselig von Hand vorgenommen werden muß und daher nicht optimiert werden kann.

Der Erfindung liegt daher die Aufgabe zugrunde, ein Verfahren und eine Vorrichtung der eingangs genannten Art dahingehend weiterzubilden, daß eine Automatisierung des Verfahrens möglich ist und weiterhin differenzierte Verfahrensschritte zur Verfügung gestellt werden, um den genannten unterschiedlichen Anforderungen gerecht zu werden.

Nach dem eingangs genannten Verfahren wird diese Aufgabe erfindungsgemäß zum einen dadurch gelöst, daß die Maische nach dem Druckentspannungsverfahren behandelt und der Druck im Behälter erfaßt und geregelt wird.

Nach dem eingangs genannten Verfahren wird die der Erfindung zugrunde liegende Aufgabe ferner dadurch gelöst, daß die Maische nach dem Druckentspannungsverfahren behandelt und der Zuckerabbau der Maische im Behälter erfaßt und in Abhängigkeit von einem gewünschten zeitlichen Verlauf des Zuckerabbaus in der Maische (Gärkurve) geregelt wird.

Nach der eingangs genannten Vorrichtung wird die Aufgabe erfindungsgemäß dadurch gelöst, daß im Behälter ein Drucksensor vorgesehen, der mit einem Steuergerät für ein Druckentspannungsventil des Behälters in Verbindung steht.

Die der Erfindung zugrundeliegende Aufgabe wird auf diese Weise vollkommen gelöst.

Während nämlich nach der herkömmlichen Vorgehensweise lediglich manuelle Stellmittel, z.B. ein Handventil, zur Verfügung standen, um den gewünschten Gärverlauf einzustellen, z.B. den Behälter manuell zu entspannen, steht jetzt ein automatisiertes Verfahren bzw. eine automatisierte Vorrichtung zur Verfügung, um über eine elektronische Regelung die Gärung im Behälter in vorbestimmter Weise führen zu können.

Auf diese Weise wird es erstmals möglich, nicht nur den Überwachungsbedarf bei derartigen Behältern zu vermindern, es wird vielmehr auch möglich, durch entsprechend differenzierte Regelung ein Feinabstimmung der Gärung vorzunehmen, so daß unterschiedlichen Rebsorten, unterschiedlichen Jahrgängen oder auch unterschiedlichen Behandlungswünschen der Kellermeister Rechnung getragen werden kann.

Bei einer bevorzugten Ausführungsform des erfindungsgemäßen Verfahrens werden folgende Schritte eingesetzt:
- Einfüllen der unvergorenen Maische in den Behälter;
- Erfassen des sich aufbauenden Gärdruckes im Behälter;
- schnelles Entspannen des Gärdruckes beim Erreichen eines ersten, vorgegebenen niedrigeren Druck-Schwellwertes;
- vorzugsweise ein- oder mehrfaches Wiederholen der beiden letzten Schritte des Erfassens und Entspannens;
- erneutes Erfassen des sich aufbauenden Gärdruckes im Behälter; und
- schnelles Entspannen des Gärdruckes bei Erreichen eines zweiten vorgegebenen höheren Druck-Schwellwertes.

Diese Maßnahmen haben den Vorteil, daß der gewünschte Abblasdruck, nämlich der zweite vorgegebene höhere Druck-Schwellwert überraschenderweise schneller erreicht werden kann, wenn man zuvor die Maische ein- oder mehrfach bei sehr viel niedrigerem Druck entspannt. Dieses mehrfachen Vorentspannen bei niedrigerem Druck bewirkt offensichtlich eine Aktivierung der Maische, mit der Folge, daß der sich dann anschließende Gärprozeß wesentlich rascher verläuft. Die Gesamtdauer des Vorganges, d.h. die Zeit vom Gärstart bis zum Erreichen des Abblasdrucks ist deutlich kürzer als wenn man die Maische in einem einzigen Gärvorgang auf den Abblasdruck bringen würde, wie dies seither der Fall war.

Dies gelingt insbesondere dann, wenn der höhere Druck-Schwellwert etwa 3 bis 6mal so groß ist wie der niedrigere Druck-Schwellwert, insbesondere wenn der höhere Druck-Schwellwert zwischen 1,5 und 5 bar und der niedrigere Druck-Schwellwert zwischen 0,5 und 1,5 bar liegt. Weiterhin kann beim ein- bis mehrfachen Wiederholen der Schritte des Erfassens und Entspannens beim Erreichen des ersten Druck-Schwellwertes der erste Druck-Schwellwert während aufeinanderfolgender Schritte variiert, vorzugsweise erhöht werden.

All diese Maßnahmen ermöglichen es, die bereits erwähnte Aktivierung der Maische durch mehrfaches kurzes Entspannen beim niedrigeren Druck zu optimieren und damit die Gesamt-Angärzeit zu verringern.

Bei einer bevorzugten Ausgestaltung der Erfindung werden die Druck-Schwellwerte in Abhängigkeit von einem gewünschten zeitlichen Verlauf des Zuckerabbaus in der Maische (Gärkurve) eingestellt.

Diese Maßnahme hat den Vorteil, daß eine kontinuierliche Regelung des Gärungszustandes möglich ist, indem nämlich ständig der Zuckerabbau bzw. die Alkoholzunahme überwacht werden und der zeitliche Verlauf dieses Parameters an einen vorgegebenen zeitlichen Verlauf angepaßt wird, indem man den Druck entsprechend einstellt. Hierbei macht man sich die Tatsache zunutze, daß ein höherer Druck in einem Druckbehälter zu einer Verlangsamung der Gärung und ein niedrigerer Druck zu einer Beschleunigung der Gärung führt. Man kann daher durch geeignete Druckeinstellung den Gärverlauf, d.h. den zeitlichen Verlauf des Zuckerabbaus und damit auch die Kontaktzeit zwischen Traubensaft und Tresterbestandteilen steuern.

Bei einer bevorzugten Ausgestaltung des eingangs als zweites genannten Verfahrens sieht man folgende Schritte vor:
- Einfüllen der unvergorenen Maische in den Behälter;
- Erfassen des Zuckerabbaus der Maische im Behälter;
- schnelles Entspannen des Gärdruckes bei Erreichen eines ersten, vorgegebenen niedrigeren Druck-Schwellwertes; und
   vorzugsweise ein- oder mehrfaches Wiederholen des Entspannens.

Diese Maßnahme hat den Vorteil, daß die soeben geschilderte Regelung des Gärverlaufes über den Druck als Stellgröße auch durch gezieltes Druckentspannen möglich ist, so daß alle Vorteile der im Rahmen der vorliegenden Anmeldung beschriebenen Ausführungsbeispiele des kontrollierten Druckabbaus genutzt werden können.

Bei einer weiteren bevorzugten Ausgestaltung des Verfahrens wird der Zuckerabbau durch Einstellen der Temperatur der Maische geregelt.

Diese Maßnahme hat den Vorteil, daß alternativ oder zusätzlich zum geregelten Druckentspannen als weitere Stellgröße die Temperatur eingesetzt werden kann, um den Gärverlauf kontinuierlich zu regeln. Hierbei macht man sich die Tatsache zunutze, daß die Gärung bei einer höheren Temperatur erheblich schneller verläuft als bei einer niedrigeren Temperatur. Das hierbei vorzusehende Temperaturintervall braucht dabei nur wenige Grade zu betragen, es kann z.B. zwischen 22 und 32°C liegen.

Ein weiteres Ausführungsbeispiel der Erfindung zeichnet sich dadurch aus, daß die Maische nach dem Vergären durch Erfassen und Regeln des pH-Wertes behandelt wird.

Diese Maßnahme hat den Vorteil, daß man in einem weiteren geregelten Behandlungsvorgang (sog. zweite Gärung) über einen längeren Zeitraum von z.B. mehreren Wochen einen biologischen Säureabbau erreichen kann. Hierbei kann man sich die bei Ausführungsbeispielen der Erfindung ohnehin vorzusehenden Einrichtungen zunutze machen, nämlich die diversen Sensoren sowie die Programmsteuerung. Wenn man nämlich beispielsweise den pH-Wert durch einen entsprechenden Sensor laufend überwacht, so kann man ebenfalls einen vorgegebenen zeitlichen Verlauf des pH-Wertes dadurch erreichen, daß man die Temperatur der Maische als Stellgröße entsprechend einstellt.

Bei einer weiteren bevorzugen Ausführungsform des erfindungsgemäßen Verfahrens wird beim Druckentspannen der Druck stufenweise vermindert.

Diese Maßnahme steht im Gegensatz zum herkömmlichen Vorgehen, bei dem der Druck schlagartig vom Abblasdruck auf Umgebungsdruck vermindert wird. Im Gegensatz dazu sieht die genannte Ausführungsform der Erfindung vor, den Druck stufenweise zu vermindem und zwar vom bereits erwähnten Abblasdruck zu einem geringfügig oberhalb des Umgebungsdruckes liegenden Basisdruck, der zugleich die Ansprechschwelle des verwendeten Drucksensors darstellt.

Die Verwendung mehrerer Druckstufen beim Druckentspannen hat den Vorteil, daß ein weiterer Parameter zur Verfügung steht, der in geeigneter Weise variiert werden kann, um das gewünschte Ergebnis zu optimieren.

So wird bei Varianten dieser Ausführungsform des erfindungsgemäßen Verfahrens der Druck beim stufenweisen Vermindern des Druckes in vorgegebenen Druckintervallen oder in vorgegebenen Zeitintervallen vermindert. Während im erstgenannten Fall die Stufen durch Druckstufen dargestellt werden, wird im zweitgenannten Fall die stufenweise Druckverminderung dadurch bewirkt, daß jeweils für eine vorbestimmte Zeit eine Druckverminderung ermöglicht wird.

Auch in diesem Falle ist bevorzugt, wenn die Intervalle bei aufeinanderfolgenden Entspannungsschritten variierbar sind. Dies eröffnet weitere Möglichkeiten der Optimierung.

Schließlich ist in diesem Zusammenhang noch eine Ausführungsform bevorzugt, bei der zwischen den Entspannungsschritten vorbestimmte Zeitintervalle oder Druckintervalle liegen, um das zeitliche Aufeinanderfolgen der Entspannungsschritte zu definieren.

Bei einer anderen Gruppe von Ausführungsbeispielen der Erfindung wird bei Druckentspannen der Druckabfall im Behälter überwacht, das Auftreten eines Zwischenmaximums erfaßt und der Druckabbau im Verlaufe des Zwischenmaximums beendet.

Diese Maßnahme hat den Vorteil, daß der Druck im Behälter nur soweit abgelassen wird, wie dies zur Überspülung des Tresterkuchens erforderlich ist. Der Druckabbau wird dann sofort gestoppt, damit der noch verbleibende Restdruck erhalten bleibt. Dies hat wiederum den Vorteil, daß beim nachfolgenden Gären der Abblasdruck schneller erreicht wird, weil der Druckaufbau nicht vom Umgebungsdruck oder einem geringfügig darüberliegenden Basisdruck, sondern vielmehr von einem beträchtlichen Druckwert ausgeht, der unmittelbar nach Erreichen des Zwischenmaximums im Behälter noch vorhanden war. Insgesamt ergibt sich damit der Vorteil, daß mit der selben Maische wesentlich mehr Entspannungsvorgänge möglich sind, weil die Gäraktivität in der Lage ist, den Abblasdruck häufiger und auch in schnellerer Folge zu erreichen. Insgesamt wurde damit die Maischebewegung und damit die Farbstoffauslaugung deutlich verbessert.

Zum Optimieren des genannten Verfahrens kann alternativ der Druckabbau bei Erreichen eines den Maximalwert des Zwischenmaximums vorausgehenden Minimalwertes beendet werden oder beim Erreichen des Maximalwertes des Zwischenmaximums selbst oder aber es wird im Zwischenmaximum ein erster Minimalwert, ein nachfolgender Maximalwert und ein wiederum nachfolgender Wert, der gleich dem Minimalwert ist, erfaßt und der Druckabbau bei Erreichen des nachfolgenden Wertes beendet.

Diese Varianten für die Zeitwahl des Abbruchs des Druckabbaus gestatten es, je nach Maischeart den optimalen Zeitpunkt zu bestimmen, bei dem die Überflutung des Tresterkuchens stattgefunden hat. So ist beispielsweise möglich, bei einer frischen Maische, bei der sich noch kein fester Tresterkuchen gebildet hat, den Zeitpunkt relativ früh zu legen, weil die noch relativ weiche Tresterschicht schneller überspült wird, während bei einer alten Maische, die bereits einen sehr festen Tresterkuchen aufweist, der Zeitpunkt später zu legen ist, weil beim Druckentspannen mehr Zeit benötigt wird, um den festen Tresterkuchen aufzubrechen und zu überspülen. Die vorstehend geschilderten Varianten des Verfahrens können daher bei nachfolgenden Entspannungsschritten während der Behandlung ein- und derselben Maische nacheinander eingesetzt werden.

Schließlich kann bei der vorstehend genannten Gruppe von Ausführungsbeispielen noch eine gute Wirkung dadurch erzielt werden, daß ein Alarm ausgelöst wird, wenn kein Zwischenmaximum erfaßbar ist.

Diese Maßnahme hat den Vorteil, daß frühzeitig erkannt wird, wenn kein regulärer Gärprozeß abläuft.

Bei einer weiteren Gruppe von Ausführungsbeispielen der Erfindung wird ein vorbestimmter Füllstand-Grenzwert überwacht und die Druckentspannung bei Erreichen des Füllstand-Grenzwertes abgebrochen.

Diese Maßnahme hat den Vorteil, daß eine Verschmutzung des Behälters und der Behälterumgebung vermieden wird, wenn beispielsweise zu viel Maische in den Behälter eingefüllt wurde und beim Druckentspannen die Maische dann so stark aufschäumt, daß Maische über das Entspannungsventil austreten würde. Auch wird vermieden, daß aufsteigender Schaum oder aufsteigende Maischebestandteile das Entspannungsventil verstopfen und damit eine gefährliche Situation herbeiführen.

Eine gute Wirkung wird in diesem Falle dadurch erzielt, daß die Druckentspannung nach einem vorbestimmten Zeitintervall ab dem Abbrechen fortgesetzt wird.

Diese Maßnahme hat den Vorteil, daß die Maische sich beruhigen kann und die Schaumentwicklung durch den wieder ansteigenden Druck im Behälter gebremst wird, ehe ein erneuter Druckabbau eingeleitet wird.

In Weiterführung dieser Maßnahme kann nach einer vorbestimmten Zeit von Abbrüchen ein Alarm ausgelöst werden.

Diese Maßnahme hat den Vorteil, daß eine fehlerhafte Befüllung des Behälters zuverlässig erkannt wird, weil ein zu voll eingefüllter Behälter zu dem genannten Betriebszustand führen kann, daß eine reguläre Druckentspannung nicht mehr möglich ist, weil die Maische zu jedem Zeitpunkt so stark aufschäumt, daß der Füllstand-Grenzwert erreicht wird.

In der Praxis hat sich gezeigt, daß es zweckmäßig ist, die vorbestimmte Zahl gleich dem maximal zulässigen Gärdruck im Behälter, gemessen in bar, einzustellen.

Sofern eine Füllstand-Überwachung vorgesehen ist, ist es ferner vorteilhaft möglich, beim Abbrechen den erreichten Druckabfall zu messen und einen Alarm dann auszulösen, wenn der Druckabfall kleiner als ein vorbestimmter Grenzwert ist.

Auch diese Maßnahme hat den Vorteil, daß eine falsche Befüllung des Behälters erkannt werden kann, weil ein zu hoch eingefüllter Behälter den genannten Alarm auslösen wird.

Bei Ausführungsbeispielen der Erfindung mit geregelter Druckentspannung können die Druckentspannungsvorgänge vor Ablauf einer vorgegebenen Ruhezeit unterdrückt werden.

Diese Maßnahme hat den Vorteil, daß man die Kontaktzeit der Maische, d.h. die Kontaktzeit zwischen Traubensaft und Tresterbestandteilen, beliebig lange oder beliebig kurz einstellen kann. Wenn man beispielsweise die Ruhezeit bei Ausführungsbeispielen der Erfindung zwischen 15 Minuten und 25 Stunden einstellt, so bedeutet dies, daß diese Ruhezeit in jedem Fall eingehalten wird, ehe die Druckentspannung vorgenommen wird. Wenn also beispielsweise bei entsprechend schnellerer Gärung der obere Druck-Grenzwert, d.h. der Abblasdruck, schon nach einer Stunde erreicht wird, andererseits aber die vorgewählte Ruhezeit drei Stunden beträgt, so wird durch entsprechende kontinuierliche Druckeinstellung (kontinuierliches Druckablassen) der Druck im Behälter für die Dauer der vorgewählten Ruhezeit bei dem vorgewählten Abblasdruck belassen und die Druckentspannung erst nach Ablauf der Ruhezeit eingestellt. Dies gilt generell für alle Ausführungsbeispiele der vorliegenden Erfindung, bei denen man von der geregelten Druckentspannung Gebrauch macht.

Weiterhin zeichnen sich Ausführungsbeispiele der erfindungsgemäßen Vorrichtung noch dadurch aus, daß sie einen Sensor für Zuckergehalt und/oder einen Sensor für den pH-Wert umfassen.

Bei einem weiteren Ausführungsbeispiel der erfindungsgemäßen Vorrichtung umfaßt diese einen Speicher für vorgegebene und/oder sich im Betrieb einstellende Gärkurven sowie vorzugsweise für Kennwerte der Maische.

Diese Maßnahme hat den Vorteil, daß eine Erfolgskontrolle möglich ist, auch über längere Zeiträume von mehreren Jahren hinweg. Hierzu hält der Kellermeister im Speicher zunächst interessierende Parameter der angelieferten Trauben bzw. Maische fest. Dies sind insbesondere Daten über die Traubensorte, die Herkunft, den Lesezeitpunkt, die Anlieferungstemperatur und dergleichen. Diesen Trauben- bzw. Maischedaten kann nun die bei der Vergärung eingestellte bzw. sich im Betrieb eingestellt habende Gärkurve, der zeitliche Verlauf des Druckes, der Temperatur, des pH-Wertes und dergleichen im Behälter zugeordnet werden, um all diese Werte in einem Langzeitspeicher abzulegen. Wenn nun der fertige Wein nach Abschluß der Gärung oder auch nach einem oder mehreren Jahren verkostet wird, so lassen sich auch die dabei ermittelten sensorischen Merkmale abspeichern, und es ist eine Erfolgskontrolle über mehrere Jahre möglich, indem zu einem späteren Zeitpunkt ausgesagt werden kann, welche Art der individuell vorgenommenen gelenkten Gärführung nun erfolgreicher war und welche nicht.

Weitere Vorteile ergeben sich aus der Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
Fig. 1 eine äußerst schematisierte Ansicht einer Ausführungsform einer erfindungsgemäßen Vorrichtung, wie sie beim Durchführen des erfindungsgemäßen Verfahrens eingesetzt werden kann;
Fig. 2 ein erstes Druck/Zeit-Diagramm zur Erläuterung einer ersten Ausführungsform des erfindungsgemäßen Verfahrens;
Fig. 3 ein zweites Druck/Zeit-Diagramm zur Erläuterung einer zweiten Ausführungsform des erfindungsgemäßen Verfahrens;
Fig. 4 ein drittes Druck/Zeit-Diagramm zur Erläuterung einer dritten Ausführungsform des erfindungsgemäßen Verfahrens.

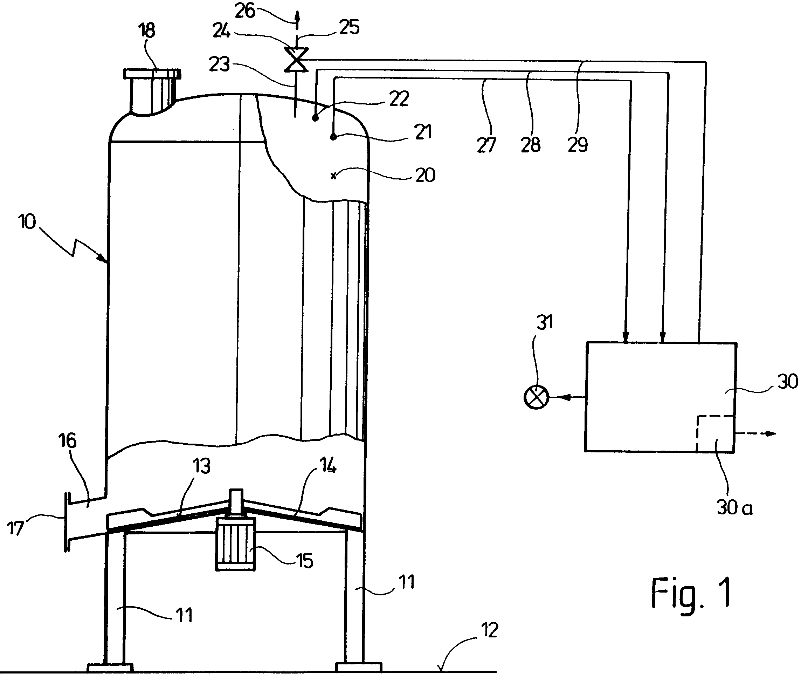

In Fig. 1 bezeichnet 10 insgesamt einen stehend-zylindrischen Behälter, der als Druckbehälter ausgebildet ist. Der Behälter 10 steht auf Stützen 11 im vertikalen Abstand über einem Fundament 12.

Ein Boden 13 des Behälters 10 ist innenkegelig ausgebildet. Ein Austragarm 14 ist mittels eines Motors 15 um eine Längsachse des Behälters 10 drehbar. Ein Austragstutzen 16 ist seitlich angesetzt und mittels eines Schiebers 17 abgeschlossen.

An der Oberseite des Behälters 10 befindet sich ein Dom 18 zum Einfüllen von Maische. Der Dom 18 ist druckdicht verschließbar.

Es versteht sich, daß der Behälter 10 lediglich als Beispiel zu verstehen ist und eine zweckmäßige Gestaltung wiedergibt. Die Erfindung kann darüberhinaus auch an anders geformten Behältern ausgeführt werden, beispielsweise in liegend-zylindrischen Behältern oder auch in Behältern, die von einer Zylinderform abweichen. Auch die Gestaltung des Behälterbodens, des Austragarms sowie des Austragstutzens sind lediglich beispielhaft zu verstehen und schränken die Erfindung nicht ein.

Im Innenraum 20 des Behälters 10 befindet sich ein Füllstandssensor 21 sowie ein Drucksensor 22. Eine Gasleitung 23 ist ebenfalls an den Innenraum 20 angeschlossen. Die Gasleitung 23 geht über ein Magnetventil 24 in eine Abblasleitung 25 über, damit Druck aus dem Innenraum 20 abgelassen werden kann, wie mit einem Pfeil 26 angedeutet.

Die Sensoren 21 und 22 sind dabei lediglich als Beispiele für Sensoren für unterschiedliche physikalische Parameter zu verstehen. Weitere Sensoren, beispielsweise Sensoren für den Zucker- bzw. Alkoholgehalt, für die Temperatur, für den pH-Wert oder dergleichen können ebenfalls vorgesehen werden; sie sind in Fig. 1 der Übersichtlichkeit halber nicht nochmals gesondert eingezeichnet worden.

Der Füllstandssensor 21, der Drucksensor 22 und entsprechend weitere eventuell vorhandene Sensoren der vorstehend genannten Art sowie das Magnetventil 24 sind jeweils über elektrische Leitungen 27, 28 und 29 mit einem elektronischen Steuergerät 30 verbunden. Das elektronische Steuergerät 30 enthält mehrere Speicher 30a und ist an eine Alarmanzeige 31 angeschlossen.

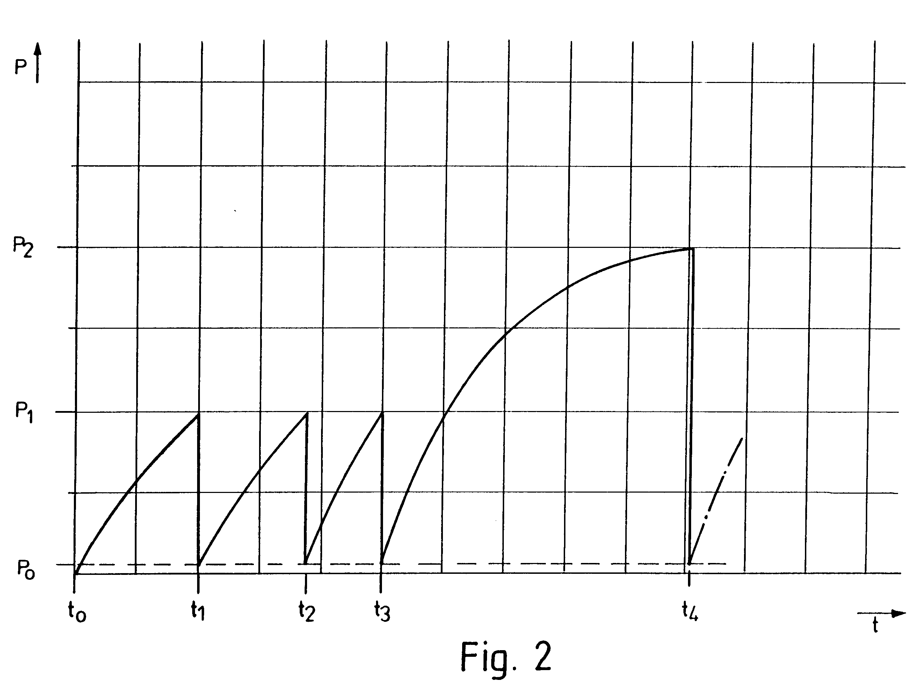

Die Wirkungsweise der Anordnung gemäß Fig. 1 soll nachstehend anhand der Diagramme gemäß den Fig. 2 bis 4 für verschiedene Ausführungsformen der Erfindung erläutert werden, die alternativ oder in Kombination miteinander eingesetzt werden können.

Es wurde bereits eingangs erwähnt, daß eine in den Druckbehälter 10 eingefüllte Maische zunächst dem Gärstart zugeführt werden muß. Bei einer kalt eingefüllten Maische geschieht dies dadurch, daß durch eine in Fig. 1 nicht dargestellte Temperiervorrichtung die Maische auf die sogenannte Gärstarttemperatur (ca. 25°C) erwärmt wird. Die nun einsetzende alkoholische Gärung der Maische führt zu einem Druckanstieg im Innenraum 20 des Behälters 10. Die alkoholische Gärung der Maische verläuft entlang von sogenannten "Gärkurven", d.h. zeitlichen Verläufen eines für die Gärung typischen Parameters, insbesondere des Zuckerabbaus bzw. der Alkoholzunahme, weil bekanntlich bei der alkoholischen Gärung der in der Maische vorhandene Zucker nach und nach in Alkohol umgewandelt wird. In der Praxis können Gärkurven, die den Zuckerabbau darstellen, im Speicher 30a entweder langsam abfallend angelegt sein, es sind aber auch Gärkurven mit zunächst schnellerem und dann langsamerem Abfallen denkbar. Ein Kerngedanke der vorliegenden Erfindung ist, dem Kellermeister für die Herstellung unterschiedlicher Weine eine größtmögliche Flexibilität für die Gärführung an die Hand zu geben, indem ihm ermöglicht wird, Gärkurven von nahezu beliebiger Art "fahren" zu können. In der vorliegenden Anmeldung ist dies als "gelenkte Gärführung" bezeichnet, wenn eine Regelung der nachstehend genannten Art eingesetzt wird.

Wie in allen Fällen der Regelung in geschlossenen Regelkreisen benötigt man für eine Regelung eine Führungsgröße, d.h. einen vorgegebenen und nachzufahrenden Verlauf, ferner eine Erfassung der Ist-Größe und schließlich einen Regler mit einem Stellglied, um die Ist-Größe durch geschlossene Regelung der Führungsgröße nachzuführen.

Bei einer Regelung der alkoholischen Gärung von Weinmaische kann man als charakteristische Größe für den Gärvorgang, wie bereits erwähnt, z.B. den Zuckerabbau, d.h. den Zuckergehalt, oder die Alkoholzunahme, d.h. den Alkoholgehalt, heranziehen. Zur Erfassung dieser Größen dienen an sich bekannte Sensoren für den Zucker- bzw. den Alkoholgehalt.

Als Führungsgröße dient die bereits erwähnte vorgegebene Gärkurve, die der Kellermeister für jeden Wein individuell festlegen kann. Als Stellgröße kann man nun entweder die Temperatur oder den Druck im Behälter einsetzen, so daß die sich ohne Beeinflussung einstellende Gärung durch entsprechendes Nachstellen der Temperatur und/oder des Drucks gerade so beeinflußt werden kann, daß sich die gewünschte Gärkurve einstellt.

Bei einem ersten Ausführungsbeispiel der Erfindung wird der Gärdruck mittels des Drucksensors 22 überwacht und im Steuergerät 30 mit dort abgelegten Kennlinien oder Wertetabellen verglichen.

Fig. 2 zeigt zum Zeitpunkt t₀ den Gärstart, der beim Umgebungsdruck einsetzt, der in der Darstellung der Fig. 2 durch den Koordinatenursprung dargestellt ist.

Der Gärdruck im Innenraum 20 steigt nun expontentiell an, bis zu einem Zeitpunkt t₁ ein erster Druck-Schwellwert p₁ erreicht ist. Dieser Schwellwert p₁ ist als Abblasdruck im Steuergerät 30 vorgegeben. Er liegt weit unter dem maximal möglichen Druck im Innenraum 20 und beträgt beispielsweise 1 bar.

Zum Zeitpunkt t₁, wenn dieser Schwellwert p₁ erreicht wurde, wird vom Steuergerät 30 ein Signal an das Magnetventil 24 geleitet und dieses verbindet die Gasleitung 23 mit der Abblasleitung 25, so daß CO₂ aus dem Innenraum 20 entweichen kann und der Druck p schlagartig abfällt, wie deutlich in Fig. 2 zu erkennen ist. Sobald der Druck p auf einen Basisdruck p₀ abgefallen ist, wird das Magnetventil 24 wieder geschlossen. Dieser Basisdruck p₀ wird so niedrig wie möglich gewählt und ist in der Praxis durch den minimalen Ansprechdruck des Drucksensors 20 bestimmt.

Als Alternative kann man eine Ruhezeit vorsehen, d.h. eine Mindestzeit, vor deren Ablauf der Abblasvorgang nicht eingestellt wird. Die Ruhezeit kann z.B. zwischen 15 Minuten und 25 Stunden betragen. Erreicht nun bei intensiver Gärung der Druck im Behälter den vorgegebenen Schwellwert p₁ vor Ablauf der Ruhezeit, so wird gleichwohl keine Druckentspannung vorgenommen, sondern es wird vielmehr der Druck im Behälter bis zum Ablauf der Ruhezeit beim Schwellwert p₁ belassen, beispielsweise indem man kontinuierlich etwas Druck abläßt, um den Überdruck wegzunehmen. Wenn die Ruhezeit dann abgelaufen ist, wird der Druck in der genannten Weise schlagartig entspannt. Auf diese Weise ist es möglich, die Kontaktzeit der Maische entsprechend einzustellen.

Der Druck p im Innenraum 20 steigt nun wieder an, bis erneut der Schwellwert p₁ erreicht ist. Dies ist zum Zeitpunkt t₂ der Fall. Nun wird das Magnetventil 24 wiederum geöffnet und der Entspannungsvorgang wiederholt sich. Der Druck p steigt nun ein drittes Mal auf den Wert p₁ an, der zum Zeitpunkt p₃ erreicht wird. Auch jetzt wird durch Öffnen des Magnetventils 24 der Innenraum 20 wieder entspannt.

Wie man aus Fig. 2 erkennen kann, beschleunigt sich der Gärvorgang dabei, so daß die Zeitintervalle zwischen t₀ und t₁ bzw. t₁ und t₂ bzw. t₂ und t₃ verkürzen. Man kann daher in einer Abwandlung des in Fig. 2 dargestellten Verlaufes den Wert p₁ für aufeinanderfolgende Entspannungsvorgänge auch variieren, beispielsweise ansteigen lassen, so daß die Zeitdauern für die einzelnen Entspannungsvorgänge wieder gleich groß oder sonstwie variiert werden.

Die vorstehend beschriebenen Schritte spielten sich, wie bereits erwähnt, bei einem sehr niedrigen Druckentspannungsniveau ab. Die Schritte dienten im wesentlichen zum Aktivieren der Maische, wie sich bereits aus den kürzer werdenden Zeitintervallen zwischen Entspannungsvorgängen ergibt.

Ab dem Zeitpunkt t₃ setzt nun die Hauptgärung ein, in deren Verlauf zum Zeitpunkt t₄ ein zweiter Druck-Schwellwert p₂ erreicht wird. Dies ist der sogenannte Abblasdruck, der zugleich in der Größenordnung des maximalen Betriebsdrucks des Behälters 10, jedoch immer noch genügend weit unterhalb des Gärdrucks des Behälters 10 liegt.

Zum Zeitpunkt t₄ wird das Magnetventil 24 wieder geöffnet und der Druck nunmehr schlagartig insgesamt bis auf den Basisdruck p₀ entspannt.

Während dieses Haupt-Entspannungsvorganges zum Zeitpunkt t₄ wird die Maische im Innenraum 20 kräftig bewegt und ein sich bereits gebildet habender Tresterkuchen vom aufschäumenden Traubensaft überspült.

Ab dem Zeitpunkt t₄ schließt sich der gleiche Vorgang noch ein- oder mehrfach an, wie er zuvor für den Zeitabschnitt zwischen t₀ und t₄ bereits dargestellt wurde. Es versteht sich dabei jedoch, daß die eingestellten Werte für nachfolgende Entspannungsvorgänge variiert werden können, um ein jeweils gewünschtes Ergebnis zu erzielen.

Festzuhalten bleibt ferner, daß die drei Vor-Entspannungsschritte zu den Zeitpunkten t₁, t₂ und t₃ eine derartige Aktivierung der Maische bewirken, daß die Gesamtzeit von t₀ bis t₄ deutlich geringer ist als bei einem herkömmlichen Entspannungsverfahren, wo der Abblasdruck p₂ durch einen einzigen, länger andauernden Druckaufbauvorgang erzielt wurde.

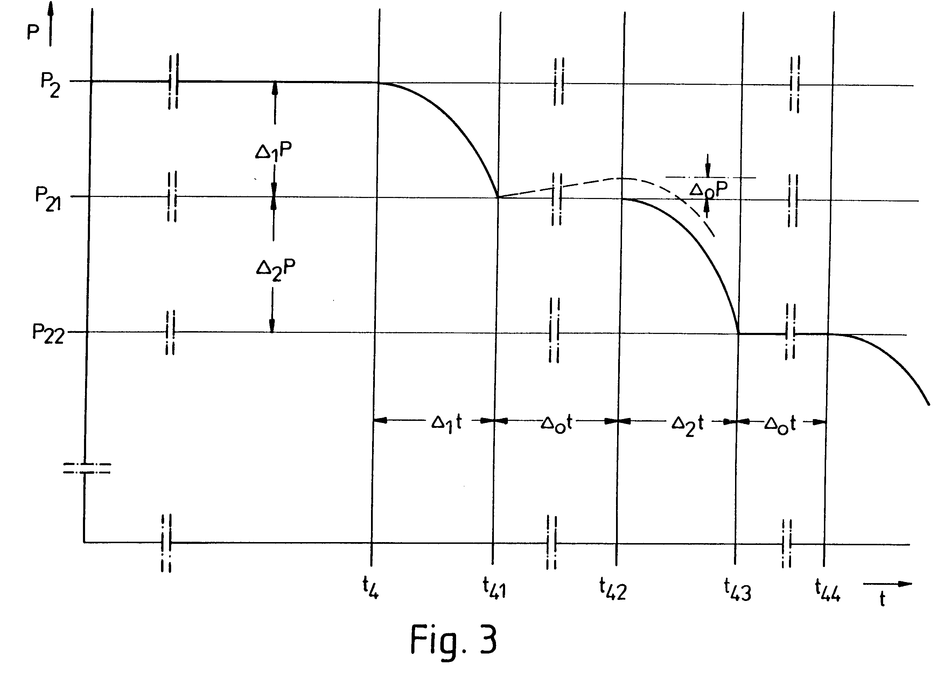

Fig. 3 zeigt einen weiteren Druck/Zeit-Verlauf, bei dem jedoch der Koordinatenursprung unterdrückt wurde und die Zeitachse um einige Größenordnungen gegenüber der Zeitachse gemäß Fig. 2 gedehnt und abschnittsweise dargestellt ist. Während nämlich bei der Darstellung der Fig. 2 der Zeitabstand zwischen t₀ und t₄ in der Größenordnung von 10 bis 12 Stunden liegt, zeigt Fig. 3 in den Zeitabschnitten jeweils nur Bereiche von Sekunden oder Millisekunden.

Betrachtet man die darstellende Fig. 3 näher, so erkennt man, daß der Druckverlauf so geführt wird, daß nach dem Erreichen des Abblasdrucks p₂ ab dem Zeitpunkt t₄ nur eine stufenweise Druckverminderung auf einen Zwischenwert p₂₁ zugelassen wird. Dies entspricht einem Druckintervall Δ ₁ₚ. Der Druckabfall nimmt dabei ein Zeitintervall Δ ₁ₜ, ein.

Zum Zeitpunkt t₄₁ wird der Druckabbau gestoppt und zwar für eine Verweilzeit Δ_{α}. Während der Verweilzeit Δ_{α} bleibt, je nachdem, wie lange diese Verweilzeit eingestellt wird, der Zwischendruck p₂₁ entweder konstant (durchgezogene Darstellung) oder er steigt wiederum an (gestrichelte Darstellung).

Nach Ablauf der Verweilzeit Δ ₀ₜ wird zum Zeitpunkt t₄₂ eine weitere stufenweise Druckentspannung vorgenommen, nämlich um ein Druckintervall Δ ₂ₚ in einem Zeitbereich Δ ₂ₜ.

Ab dem Zeitpunkt t₄₃ wird dann eine erneute Verweilzeit Δ _{α} eingestellt, und das Spiel wiederholt sich ggf. noch mehrfach. Zur Durchführung des in Fig. 3 veranschaulichten Druckstufenverfahrens kann entweder jeweils das Druckintervall Δ ₁ₚ, Δ ₂ₚ ... oder das zugehörige Zeitintervall Δ ₁ₜ, Δ ₂ₜ ... vorgegeben werden, um die Druckstufen zu definieren.

Die Verweilzeiten Δ ₀ₜ können entweder als Zeitwerte oder bei zwischenzeitlich ansteigendem Druck (gestrichelter Verlauf zwischen t₄₁ und t₄₂) als Druckwert Δ ₀ₚ vorgegeben werden.

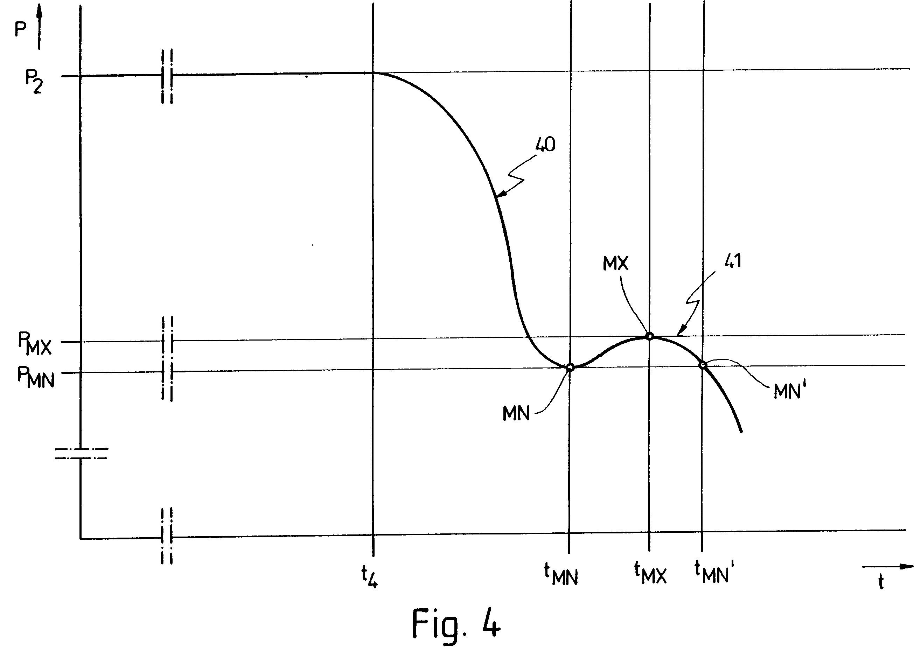

Fig. 3 zeigt eine weitere Variante beim Druckabbau, wobei die Achsmaßstäbe in Fig. 4 weitgehend denjenigen der Fig. 3 entsprechen.

Aus Fig. 4 ist zu erkennen, daß der Druckverlauf vom Abblasdruck p₂ dann, wenn man das Magnetventil 24 zum Zeitpunkt t₄ öffnet, entlang eines Druckabfalls 40 abfällt.

Erstaunlicherweise ist nun der Druckabfall 40 nicht kontinuierlich, er stellt sich vielmehr im Druckablauf 40 ein Zwischenmaximum 41 ein.

Dieses Zwischenmaximum 41 manifestiert sich zunächst durch einen Minimalwert MN, der zum Zeitpunkt t_{MN} erreicht wird, einen darauffolgenden Maximalwert MX zum Zeitpunkt t_{mX} sowie einen wiederum darauffolgenden Zwischenwert MN′ zum Zeitpunkt t_{MN′}, wobei der letztgenannte Wert MN′ dem Minimalwert MN in seiner Amplitude entspricht.

Die zugehörigen Maximal- und Minimal-Druckwerte sind in Fig. 4 mit p_{MX} bzw. p_{MN} bezeichnet.

Der Grund für das Auftreten des Zwischenmaximums 41 ist folgender:
Wenn zum Zeitpunkt t₄ in Fig. 4 das Abblasventil 24 geöffnet wird, stellt sich der Druckabfall 40 ein. Dieser vermindert sich jedoch nicht stetig, wie aus Fig. 4 deutlich erkennbar ist. Durch den abfallenden Druck wird vielmehr CO₂ im Traubensaft freigesetzt, der sich zunächst unterhalb des Tresterkuchens ansammelt und diesen dann schlagartig durchbricht. Dieses plötzliche Freiwerden einer größeren Menge von CO₂, verbunden mit einem Druchbrechen und Überspülen des Tresterkuchens drückt sich durch das Maximum MX in Fig. 4 aus, d.h. einen vorübergehenden Wieder-Anstieg des Drucks, ehe dieser nach erfolgtem Überspülen des Tresterkuchens über den Punkt MN′ hinaus kontinuierlich absinkt.

Den Verlauf des Drucks in Fig. 4 im Bereich des Zwischenmaximums 41 kann man in einfacher Weise dadurch erfassen, daß in kurzen Zeitabständen die Werte des Drucks p gemessen und jeweils mit dem vorhergehenden Wert verglichen werden. So wird zunächst nach dem ersten Druckabfall 40 eine Abnahme aufeinanderfolgender Meßwerte festgestellt werden. Sobald dann jedoch das Minimum MN durchlaufen wurde, wird der erste, nachfolgende Meßwert wieder einen Anstieg, d.h. eine positive Differenz aufzeigen. Dies ist (unter Berücksichtigung gewisser Schwellwerte zum Unterdrücken von Störungen) ein Indiz dafür, daß der vorhergehende Meßwert das Minimum MN war. Es kann somit bereits einen Meßwert nach dem Minimum MN ein Abbruch des Druckabfalls durch Schließen des Abblasventils 24 bewirkt werden. Entsprechendes gilt für das darauffolgende Maximum MX. Der Wert MN′ kann in einfacher Weise dadurch ermittelt werden, daß die nach dem Durchlaufen des Maximums MX erfaßten Meßwerte mit dem Wert des Minimums MN verglichen werden und bei Gleichheit ein Steuersignal für die Betätigung des Abblasventils 24 ausgelöst wird.

Die Frage, ob der Druckabbau im Punkt MN im Punkt MX oder im Punkt MN′ abgebrochen werden soll, kann u.a. davon abhängen, ob sich bei der Maische im Behälter 10 um eine junge Maische oder um eine alte Maische handelt. Unter "junger"Maische soll dabei ein solche verstanden werden, die sich gerade am Beginn des Gärprozesses befindet und bei der infolgedessen die Festbestandteile der Maische noch nicht oder nur unwesentlich zu einem Tresterkuchen verbacken sind. Unter "alter" Maische soll demgegenüber eine Maische verstanden werden, bei der die Tresterbestandteile bereits zu einem festen Tresterkuchen verbacken sind.

Wäre nun bei einer jungen Maische der Druckabbau eher, d.h. im Punkt MN abgebrochen werden kann, weil die Überspülung der noch losen Tresterbestandteile früher stattfindet, muff bei einer älteren Maische länger zugewartet werden, d.h. bis zum Punkte MX oder gar bis zum Punkte MN′.

Es versteht sich, daß der in Fig. 4 dargestellte Verlauf auch mehrfach nacheinander, entsprechend dem Verlauf der Fig. 3, eingestellt werden kann. Sämtliche Werte sind dabei wiederum variabel bzw. vom speziellen Druckverlauf bzw. Gärverlauf abhängig.

Bei dem erfindungsgemäßen Verfahren und der erfindungsgemäßen Vorrichtung sind ferner einige Sicherheitsmaßnahmen vorgesehen. Diese Sicherheitsmaßnahmen beziehen sich im wesentlichen auf eine Füllstand-Kontrolle, mit der sichergestellt werden soll, daß es nicht zu einem Überlauf des Behälters 10 kommt. Zu diesem Zwecke ist der Füllstandssensor 21 vorgesehen. Der Füllstandssensor 21 ist von einer Art, daß er nicht nur beim Übergang von einem gasförmigen in ein flüssiges Medium anspricht, sondern vielmehr auch erkennt, ob ein Schaum in seinen Bereich gelangt. In beiden Fällen, d.h. im Fall einer vollständigen wie auch einer unvollständigen Benetzung des Füllstandssensors wird ein entsprechendes Steuersignal ausgelöst.

In diesem Zusammenhang ist zu berücksichtigen, daß mitunter in Kellereien der Fehler begangen wird, Behälter mir mehr Maische zu befüllen als dies an sich dem höchsten zulässigen Füllstand entspricht. Mitunter geschieht dies deswegen, weil die letzte angelieferte Traubenmenge nur etwas weniger als die maximal zulässige Füllmenge eines Behälters ausmacht und man daher versucht ist, diese Mehrmenge noch in dem Behälter unterzubringen, anstatt einen weiteren Behälter hinzuzunehmen.

Insoweit bleibt dabei gelegentlich außer Acht, daß Behälter, in denen eine Maischegärung stattfindet, stets genügend Gärraum oberhalb der frisch eingefüllten Maische benötigen, damit sich die Maische beim Gären entsprechend ausdehnen kann und Druckentspannungsprozesse störungsfrei ablaufen können.

Der Füllstandssensor 21 ist nun so im Behälter 10 gemäß Fig. 1 angeordnet, daß er deutlich oberhalb des Spiegels von frisch eingefüllter Maische liegt. Erst beim nachfolgenden Gären oder beim Druckentspannen kann dann eine Situation eintreten, bei der der Füllstandssensor 21 mit Maische benetzt wird und entsprechend anspricht.

Wenn z.B. bei irgendeinem der vorstehend beschriebenen Verfahren eine Druckentspannung durch Öffnen des Abblasventils 24 bewirkt werden soll und sich dann die Maische derart nach oben bewegt, daß der Füllstandssensor 21 benetzt wird, so bewirkt das Steuergerät 30, daß das Abblasventil 24 sofort wieder schließt und der Druckentspannungsvorgang damit abgebrochen wird. Es baut sich dann nämlich sofort wieder ein Druck im Innenraum 20 auf, der das Aufschäumen der Maische beendet, so daß der Füllstandssensor 21 sich wieder außerhalb der Maische befindet.

Um nun zu erkennen, ob es sich dabei um eine eher zufällige oder um eine systematische Störung handelt, wird nach einer vorbestimmten Zeitdauer, beispielsweise nach einigen Minuten, das Abblasventil 24 wieder geöffnet und ein erneuter Druckentspannungsvorgang eingeleitet.

Wenn dann der Aufschäumvorgang der Maische wieder so stürmisch verläuft, daß der Füllstandssensor 21 benetzt wird, wiederholt sich die bereits erläuterte Sicherheitsmaßnahme und das Abblasventil 24 schließt wieder.

Es kann nun eine Zählung dieser Sicherheitsabschaltungen vorgenommen werden und ein Alarm wird dann ausgelöst, wenn mehr als eine bestimmte Anzahl von Abschaltungen vorgenommen wird. Diese Anzahl hängt zweckmäßigerweise vom vorgegebenen Abblasdruck in numerisch einfach bestimmbarer Weise ab. So hat es sich z.B. als zweckmäßig erwiesen, bei einem Abblasdruck von 3 bar insgesamt 3 Abschaltvorgänge vorzusehen, bei einem Abblasdruck von 4 bar insgesamt 4 Abschaltvorgänge usw.. Es können aber hier entsprechende Sicherheitszuschläge noch durch Addieren konstanter Werte eingearbeitet werden.

Eine weitere Kontrollmaßnahme besteht darin, daß beim Abbruch eines Druckentspannungsvorganges der zuvor erreichte Druckabfall gemessen wird. Wenn es sich dabei nur um einen sehr geringen Druckdifferenz-Betrag handelt, so liegt vermutlich ebenfalls eine zu große Befüllung des Behälters vor, weil die sehr geringe Druckdifferenz darauf hinweist, daß sich zuviel Maische im Behälter befindet und das Ansprechen des Füllstandssensors 21 beim Druckentspannen weniger durch das Druckentspannen selbst als durch den zu hohen Füllstand verursacht wurde.

Es versteht sich, daß darüberhinaus auch noch weitere Sicherungsmaßnahmen vorgesehen werden können.

So kann beispielsweise mittels des Drucksensors 22 überwacht werden, ob sich innerhalb eines vorbestimmten Zeitintervalls beim Angären der Maische auch ein bestimmter Gärdruck aufbaut. Falls dieses nicht der Fall ist, deutet dies darauf hin, daß entweder ein Fehler beim Befüllen des Behälters begangen wurde, daß die Maischetemperierung (Erwärmen auf Gärstarttemperatur) fehlerhaft arbeitet oder daß sonstige Störungen im System vorhanden sind.

Ferner kann beim Verfahren, wie dies anhand der Fig. 4 erläutert wurde, festgestellt werden, ob überhaupt ein Zwischenmaximum 41 auftritt oder nicht. Da es bei ordnungsgemäß befülltem Behälter 10 zu einem solchen Zwischenmaximum 41 unter normalen Umständen kommen muß, ist ein Ausbleiben des Zwischenmaximums 41 entweder ein Hinweis darauf, daß der Gärvorgang nicht ordnungsgemäß abläuft oder daß eine Störung im System vorliegt.

Es versteht sich ferner, daß der Behälter 10 auch mit einem Rührwerk gekoppelt sein kann, um die Auslaugung der Maische zu unterstützen. Das Rührwerk arbeitet zweckmäßigerweise so, daß es eine bestimmte Zeit in einer Richtung und eine bestimmte Zeit in der entgegengesetzten Richtung gedreht wird und sich dann eine Ruhezeit anschließt. Die Rührzeit beträgt dabei vorzugsweise einige Minuten und die Ruhezeit mindestens das 10-fache davon, typischerweise eine Stunde oder mehr.

Es versteht sich schließlich, daß beim erfindungsgemäßen Verfahren übliche Temperiertechniken eingesetzt werden können und daß der Behälter 10 im übrigen im Hinblick auf die Maischeauslaugung und das Austragen der Tresterbestandteile hin optimiert ist, wie dies jedoch an sich im Stand der Technik bekannt ist.

Es wurde bereits eingangs erwähnt, daß die Gärführung nach der vorliegenden Erfindung nicht nur durch Beeinflussung des Drucks als Stellgröße sondern auch durch Beeinflussen der Temperatur vorgenommen werden kann. Wenn man demzufolge alle erforderlichen Sensoren, eine programmierbare Steuerung und den Druck oder die Temperatur als Stellgröße zur Verfügung hat, lassen sich mit denselben Gerätschaften auch weitere, der eigentlichen Gärung nachfolgende Verfahren durchführen.

Hier sei insbesondere der biologische Säureabbau von ausgegorenen Weinen erwähnt. Beim biologischen Säureabbau muß der pH-Wert entsprechend eingestellt werden. Dies kann man durch Temperierung der ausgegorenen Maische erreichen. Auch hier verwendet man also wieder einen geschlossenen Regelkreis, bei dem der augenblickliche pH-Wert gemessen und mit einem vom Kellermeister individuell vorgegebenen zeitlichen Verlauf des pH-Wertes verglichen wird. Liegt eine Differenz zwischen Ist-Wert und Soll-Wert vor, so kann der pH-Wert durch Variation der Temperatur als Stellgröße nachgestellt werden. Diese sogenannte zweite Gärung kann über mehrere Wochen andauern.

## Patentansprüche

1. Verfahren zum Behandeln von Maische, insbesondere Rotwein-Maische, bei dem die Maische in einen Behälter (10) eingefüllt, mindestens ein physikalischer Parameter der Maische erfaßt und ein Gärverlauf der Maische in Abhängigkeit von dem Parameter geregelt wird, dadurch gekennzeichnet, daß die Maische nach dem Druckentspannungsverfahren behandelt und der Druck (p) im Behälter (10) erfaßt und geregelt wird.

2. Verfahren nach Anspruch 1, gekennzeichnet durch die Schritte:
- Einfüllen der unvergorenen Maische in den Behälter (10);
- Erfassen des sich aufbauenden Gärdruckes (p) im Behälter (10);
- schnelles Entspannen des Gärdruckes (p) beim Erreichen eines ersten, vorgegebenen niedrigeren Druck-Schwellwertes (p₁);
- vorzugsweise ein- oder mehrfaches Wiederholen der beiden letzten Schritte des Erfassens und Entspannens;
- erneutes Erfassen des sich aufbauenden Gärdruckes (p) im Behälter (10) und
- schnelles Entspannen des Gärdruckes (p) bei Erreichen eines zweiten, vorgegebenen höheren Drück-Schwellwertes (p₂).

3. Verfahren nach Anspruch 2, dadurch gekennzeichnet, daß der höhere Druck-Schwellwert (p₂) etwa zwei- bis sechsmal so groß ist wie der niedrigere Druck-Schwellwert (p₁) und daß der höhere Druck-Schwellwert (p₂) vorzügsweise zwischen 1,5 und 5 bar und der niedrigere Druck-Schwellwert (p₁) zwischen 0,5 und 1,5 bar liegt.

4. Verfähren nach Anspruch 2 öder 3, dadurch gekennzeichnet, daß beim ein- bis mehrfachen Wiederholen der Schritte des Erfassens und Entspannens beim Erreichen des ersten Druck-Schwellwertes (p₁) der erste Druck-Schwellwert während aufeinanderfolgender Schritte variiert, vorzugsweise erhöht wird.

5. Verfahren nach Anspruch 2, dadurch gekennzeichnet, daß die Druck-Schwellwerte (p₁, p₂) in Abhängigkeit von einem gewünschten zeitlichen Verlauf des Zuckerabbaus in der Maische (Gärkurve) eingestellt werden.

6. Verfahren zum Behandeln von Maische, insbesondere Rotwein-Maische, bei dem die Maische in einen Behälter (10) eingefüllt, mindestens ein physikalischer Parameter der Maische erfaßt und ein Gärverlauf der Maische in Abhängigkeit von dem Parameter geregelt wird, dadurch gekennzeichnet, daß die Maische nach dem Druckentspannungsverfahren behandelt und der Zuckerabbau der Maische im Behälter (10) erfaßt und in Abhängigkeit von einem gewünschten zeitlichen Verlauf des Zuckerabbaus in der Maische (Gärkurve) geregelt wird.

7. Verfahren nach Anspruch 6, gekennzeichnet durch die Schritte:
- Einfüllen der unvergorenen Maische in den Behälter (10);
- Erfassen des Zuckerabbaus der Maische im Behälter (10);
- schnelles Entspannen des Gärdruckes (p) beim Erreichen eines ersten, vorgegebenen niedrigeren Druck-Schwellwertes (p₁) und
- vorzugsweise ein- oder mehrfaches Wiederholen des Entspannens.

8. Verfahren nach Anspruch 6 oder 7, dadurch gekennzeichnet, daß der Zuckerabbau durch Einstellen der Temperatur der Maische geregelt wird.

9. Verfahren nach einem oder mehreren der Ansprüche 1 bis 8, dadurch gekennzeichnet, daß die Maische nach dem Vergären durch Erfassen und Regeln des pH-Wertes behandelt wird.

10. Verfahren nach einem oder mehreren der Ansprüche 1 bis 9, dadurch gekennzeichnet, daß beim Druckentspannen der Druck (p) stufenweise (p₂₁, p₂₂) vermindert wird, wobei der Druck (p) vorzugsweise in vorgegebenen Druckintervallen (Δ p) oder in vorgegebenen Zeitintervallen (Δ t) vermindert wird.

11. Verfahren nach Anspruch 10, dadurch gekennzeichnet, daß die Intervalle (Δ p, Δ t) bei aufeinanderfolgenden Entspannungsschritten variierbar sind.

12. Verfahren nach Anspruch 10 oder 11, dadurch gekennzeichnet, daß zwischen den Entspannungsschritten vorbestimmte Zeitintervalle (Δ ₀t) oder Druckintervalle (Δ ₀p) liegen.

13. Verfahren nach einem oder mehreren der Ansprüche 1 bis 12, dadurch gekennzeichnet, daß beim Druckentspannen der Druckabfall (40) im Behälter (10) überwacht, das Auftreten eines Zwischenmaximums (41) erfaßt und der Druckabbau im Verlaufe des Zwischenmaximums (41) beendet wird, wobei entweder der Druckabbau bei Erreichen eines dem Maximalwert (MX) des Zwischenmaximums (41) vorausgehenden Minimalwertes (MN) oder bei Erreichen eines Maximalwertes (MX) des Zwischenmaximums (41) beendet wird, oder im Zwischenmaximum (41) ein erster Minimalwert (MN), ein nachfolgender Maximalwert (MX) und ein wiederum nachfolgender Wert (MN′), der gleich dem Minimalwert (MN) ist, erfaßt und der Druckabbau bei Erreichen des nachfolgendes Wertes (MN′) beendet wird.

14. Verfahren nach Anspruch 13, dadurch gekennzeichnet, daß ein Alarm (31) ausgelöst wird, wenn kein Zwischenmaximum (41) erfaßbar ist.

15. Verfahren nach einem oder mehreren der Ansprüche 1 bis 14, dadurch gekennzeichnet, daß ein vorbestimmter Füllstand-Grenzwert überwacht und die Druckentspannung bei Erreichen des Füllstands-Grenzwertes abgebrochen wird, wobei vorzugsweise die Druckentspannung nach einem vorbestimmten Zeitintervall ab dem Abbrechen fortgesetzt wird, insbesondere nach einer vorbestimmten Zahl von Abbrüchen ein Alarm (31) ausgelöst wird, und beispielsweise die vorbestimmte Zahl gleich dem maximal zulässigen Gärdruck (p) im Behälter (10), gemessen in bar, ist.

16. Verfahren nach Anspruch 15, dadurch gekennzeichnt, daß beim Abbrechen der erreichte Druckabfall gemessen und ein Alarm (31) ausgelöst wird, wenn der Druckabfall kleiner als ein vorbestimmter Grenzwert ist.

17. Verfahren nach einem oder mehreren der Ansprüche 1 bis 16, dadurch gekennzeichnet, daß Druckentspannungsvorgänge vor Ablauf einer vorgegebenen Ruhezeit unterdrückt werden.

18. Verfahren nach Anspruch 17, dadurch gekennzeichnet, daß die Ruhezeit zwischen 15 Minuten und 25 Stunden liegt.

19. Vorrichtung zum Durchführen des Verfahrens nach einem oder mehreren der Ansprüche 1 bis 18, dadurch gekennzeichnet, daß im Behälter (10) ein Drucksensor (22) vorgesehen ist, der mit einem Steuergerät (30) für ein Druckentspannungsventil (24) des Behälters (10) in Verbindung steht.

20. Vorrichtung zum Durchführen des Verfahrens nach einem oder mehreren der Ansprüche 1 bis 18, dadurch gekennzeichnet, daß im Behälter (10) ein Sensor für den Zuckergehalt oder Alkoholgehalt vorgesehen ist, der mit einem Steuergerät (30) des Behälters (10) in Verbindung steht.

21. Vorrichtung zum Durchführen des Verfahrens nach einem oder mehreren der Ansprüche 1 bis 18, dadurch gekennzeichnet, daß im Behälter (10) ein Sensor für den pH-Wert vorgesehen ist, der mit einem Steuergerät (30) des Behälters (10) in Verbindung steht.

22. Vorrichtung nach einem oder mehreren der Ansprüche 19 bis 21, dadurch gekennzeichnet, daß sie einen Speicher (30a) für vorgegebene und/oder sich im Betrieb einstellende Gärkurven sowie vorzugsweise für Kennwerte der Maische umfaßt.
